# EUROPEAN PATENT SPECIFICATION

(11) **EP 2 535 061 B1**
(45) Date of publication and mention of the grant of the patent: **16.07.2014**
(21) Application number: 11170083.7
(22) Date of filing: 16.06.2011
(51) Int. Cl.: A61L 15/42, A61L 15/20, A61L 15/26, A61L 15/40

(54) **Cooling composition and absorbent article comprising the same**
Kühlungszusammensetzung und saugfähiger Artikel damit
Composition de refroidissement et article absorbant la comprenant

(43) Date of publication of application: 19.12.2012
(73) Proprietor: The Procter & Gamble Company, Cincinnati, OH 45202 (US)
(72) Inventor: Gagliardini, Alessandro, 65010 Pescara (IT)
(74) Representative: Briatore, Andrea

(56) References cited:
- US-A1- 2002 120 242
- US-A1- 2004 082 928
- US-A1- 2005 266 057

## Description

### FIELD OF THE INVENTION

The present invention relates to compositions comprising a cooling agent having improved stability and to absorbent articles comprising such compositions. The compositions are able to provide long lasting cooling effect when applied to the skin and/or mucosal membranes of a mammal, preferably human, without the need of modifying body surface temperature.

### BACKGROUND OF THE INVENTION

Absorbent articles comprising compositions which include a cooling agent such as menthyl lactate are known in the art. Such absorbent articles are able to provide a cooling sensation to the wearer without compromising on the safety profile towards the skin and/or mucosal membrane or even improving it.

However, the problem with the compositions of the prior art is that the cooling agent may have a tendency to vaporize and evaporate at room temperature. This may occur before the use of the articles, especially during the storage of absorbent articles in warehouses wherein the articles may sometimes be subjected to high temperatures. In such a case, the articles may not be able to offer the intended functionality even after a short period of storage.

In order to overcome this inconvenient, absorbent articles comprising a cooling agent have been individually packaged in a packaging impervious to the vapors of cooling agent in order to reduce the risk of release of the cooling agent from the articles before the use of the articles. However, the use of individual packaging considerably increases the manufacturing costs of such articles.

Therefore, there is a need to provide a composition comprising a cooling agent which is able to retain the cooling agent inside an absorbent article before use in a cost effective-manner.

The inventors have now found a way to solve the problem by providing a composition which comprises a cooling agent and a polyethylene glycol (PEG) or a mixture of different polyethylene glycols having high molecular weights.

### SUMMARY OF THE INVENTION

The present invention relates to a composition comprising:
a) a cooling agent, wherein the cooling agent is selected from the group consisting of menthol derivatives group consisting of method, menthyl lactate, menthone glycerin acetal and combinations thereof; and
b) a polyethylene glycol or a mixture of different polyethylene glycols, wherein the polyethylene glycol(s) has/have a weight average molecular weight M_{w} of 5000 to 40000 Da.

The present invention also relates to an absorbent article comprising such a composition.

### DETAILED DESCRIPTION OF THE INVENTION

The term "cooling agent" is used herein to refer to an agent which is able to convey to a mammal, preferably human, a freshness sensation (also called herein cooling sensation) when topically applied to said mammal/human. The cooling agent may be a chemical compound.

The term "absorbent article" is used herein to refer to devices that absorb and contain body exudates, and, more specifically, refers to devices that are placed against or in proximity to the body of a wearer to absorb and contain the various exudates discharged from the body. Absorbent articles include diapers, training pants, adult incontinence undergarments, feminine hygiene products and the like. As used herein, the term "body fluids" or "body exudates" includes, but is not limited to, urine, blood, vaginal discharges, breast milk, sweat and fecal matter.

### The Cooling agent(s)

The cooling agents suitable for use herein include all cooling agents being able to penetrate skin barrier and for which the cooling effect (also called herein freshness effect) is a physiological effect due to the direct action of these agents on the nerve endings of the body responsible for the detection of cold without any occurrence of temperature change on the surface of the body. It is believed that these agents act as a direct chemical stimulus on the cold receptors at the nerve endings, which in turn stimulate the central nervous system. In this way a freshness/cooling sensation is simulated even in absence of real change in skin temperature. Due to the persistence of the stimulus, a long lasting freshness/cooling sensation is delivered even after removal of the cooling agent. It is to be understood herein that the freshness/cooling sensation is personnel to a given individual. It must be admitted that skin tests are somewhat subjective, some individuals experiencing a greater or lesser freshness/cooling sensation than others when subjected to the same test. This perception depends on the density of thermo-receptors on skin and on the skin thickness. Typically it is observed that the thinner the skin is the more intense is the cooling sensation (also called herein freshness sensation). Without to be bound by any theory, it is believed that the thinner the skin is, the more rapid is the penetration of the cooling agent through the skin and higher is the absorption level thereof. Furthermore, studies have demonstrated that geographic factors and/or races further play a role in perception of freshness sensation.

The cooling agents that are suitable for use in the composition of the present invention encompasse the cooling agents which are disclosed in US 2005/0203473A1.

The cooling agent which is comprised in the composition of the present invention is selected from the menthol derivatives group consisting of menthol, menthyl lactate, menthone glycerin acetal and combinations thereof.

### Polyethylene glycol (PEG)

The composition of the present invention also comprises a polyethylene glycol or a mixture of different polyethylene glycols having a high molecular weight. The polyethylene glycol(s) has/have a weight average molecular weight M_{w} of 50000 Da to 40000 Da or of 10000 to 25000 Da, or of 10000 to 20000 Da.

PEGs are typically used in the composition as a carrier vehicle for delivering an effective concentration of a cooling agent to a wearer's skin but since PEGs are also emollients, they are particularly beneficial to skin, they improve skin hydration and softness, and hence maintain or even improve skin health. PEGs assure a film-forming capacity on the skin, which gives emolliency and helps prevent skin dehydration when directly contacting the skin, thereby reducing or even eliminating the occurrence of skin itching or burning. PEGs are also able to locate themselves between the layers of the epidermis (thanks to their similarity with substances naturally contained in the epidermis (stratum corneum)), enhancing thereby the elastic properties of the skin.

PEGs having a weight average molecular weight of 2000 Da or more are not only acting as a carrier vehicle for delivering the cooling agent to the skin but also help to increase the stability of the cooling agent in the composition, i.e. to retain the cooling agent into the composition and therefore reduce the risk that the cooling agent may vaporize and evaporate at room temperature.
Without being bound to theory, it is speculated that once the cooling agent is trapped into the PEGs matrix its evaporation is inhibited also for physical reasons as the cooling agent remain entrapped within the molecular structure of the PEGs. Higher weight average molecular weight PEGs have a higher entrapment effect.

In some embodiments, the weight percentage of cooling agent present in the composition is of 1 to 99 wt%, or of 10 to 70 wt%, or of 30 to 60 wt%.

### The absorbent article

In some embodiments, the absorbent article of the present invention comprises a topsheet, a backsheet and an absorbent core positioned between the topsheet and the backsheet.

The tospheet is typically directly facing the wearer in use, and the backsheet directly facing the garment in use, and the absorbent core sandwiched there between.

### The absorbent core

The absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components. According to the present invention the absorbent may have any thickness depending on the end use envisioned.

### (a) Primary/Secondary Fluid Distribution Layer

One optional component of the absorbent core of the absorbent article according to the present invention is a primary fluid distribution layer and a secondary fluid distribution layer. The primary fluid distribution layer typically underlies the topsheet and is in fluid communication therewith. The topsheet transfers the acquired fluid to this primary fluid distribution layer for ultimate distribution to the storage layer. This transfer of fluid through the primary fluid distribution layer occurs not only in the thickness, but also along the length and width directions of the absorbent article. In some embodiments, the also optional secondary fluid distribution layer typically underlies the primary fluid distribution layer and is in fluid communication therewith. The purpose of this secondary fluid distribution layer is to readily acquire fluid from the primary fluid distribution layer and transfer it rapidly to the underlying storage layer. This helps the fluid capacity of the underlying storage layer to be fully utilized. The fluid distribution layers can be comprised of any material typical for such fluid distribution layers. In particular fibrous layers which maintain the capillaries between fibers even when wet are useful as fluid distribution layers.

### (b) Fluid Storage Layer

Positioned in fluid communication with, and typically underlying the primary or secondary distribution layers, is a fluid storage layer. The fluid storage layer typically comprises any usual absorbent gelling materials. It preferably comprises such materials in combination with suitable carriers.

Suitable carriers include materials, which are conventionally utilized in absorbent structures such as natural, modified or synthetic fibers, particularly modified or non-modified cellulose fibers, in the form of fluff and/or tissues. In some embodiments, suitable carriers are tissues or tissue laminates in the context of sanitary napkins and panty liners.

In some embodiments, the fluid storage layer comprises multiple layers comprising a double layer tissue laminate typically formed by folding the tissue onto itself. These layers can be joined to each other for example by adhesive or by mechanical interlocking or by hydrogen bridge bands. Absorbent gelling materials and/or other optional materials can be comprised between the layers.

In some embodiments, the fluid storage layer comprises modified cellulose fibers such as the stiffened cellulose fibers. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The fluid storage layer can also comprise filler materials, such as Perlite, diatomaceous earth, Vermiculite, etc., to improve liquid retention.

If the absorbent gelling materials are dispersed non-homogeneously in a carrier, the fluid storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### (c) Optional Fibrous ("Dusting") Layer

An optional component for inclusion in the absorbent core is a fibrous layer adjacent to, and typically underlying the fluid storage layer. This underlying fibrous layer is typically referred to as a "dusting" layer since it provides a substrate on which to deposit absorbent gelling materials in the storage layer during manufacture of the absorbent core. Indeed, in those instances where the absorbent gelling materials are in the form of macro structures such as fibers, sheets or strips, this fibrous "dusting" layer need not be included. However, this "dusting" layer provides some additional fluid-handling capabilities such as rapid wicking of fluid along the length of the pad.

### (d) Other Optional Components of the absorbent structure

The absorbent core can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

### The topsheet

The absorbent article comprises a topsheet. The topsheet may comprise a single layer or a multiplicity of layers.

The topsheet as a whole and hence each layer individually needs to be compliant, soft feeling, and non-irritating to the wearer's skin. It also can have elastic characteristics allowing it to be stretched in one or two directions. In some embodiments, the topsheet is formed from any of the materials available for this purpose and known in the art, such as woven and non woven fabrics and films.

In some embodiments, the topsheet comprises an upper layer which provides the user facing surface of the topsheet and a lower layer (also called secondary topsheet) between the upper layer and the absorbent core.

In some embodiments, the topsheet is an apertured topsheet which comprises a plurality of apertures having a pore size of 0.0001 mm to 5 mm. All apertures might have the same dimensions or apertures of different dimensions might be present. The open area of the apertured topsheet is typically from 1% to 50%, or from 5% to 45%, or from 10% to 40%, or from 20% to 35%.

In such embodiments, the apertured topsheet typically comprises an apertured polymeric film. Suitable apertured polymeric films for use herein include polymeric apertured formed films, apertured formed thermoplastic films, apertured plastic films, and hydroformed thermoplastic films; porous foams; reticulated foams; reticulated thermoplastic films; and thermoplastic scrims.

In some embodiments, the topsheet comprises an apertured formed film. Apertured formed films are particularly suitable for use herein because they are pervious to body exudates and yet non-absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135 (Thompson), issued December 30, 1975; U.S. Patent 4,324,246 (Mullane, et al.), issued April 13, 1982; U.S. Patent 4,342,314 (Radel. et al.), issued August 3, 1982; U.S. Patent 4,463,045 (Ahr et al.), issued July 31, 1984; and U.S. 5,006,394 (Baird), issued April 9, 1991. Particularly preferred microapertured formed film topsheets are disclosed in U.S. patent 4,609,518 (Curro et al), issue September 2, 1986 and U.S. patent 4,629,643 (Curro et al), issued December 16, 1986.

Other suitable apertured topsheet for use herein are made of woven or nonwoven materials or knit materials. Such materials might be made of natural fibers (e.g., wood or cotton fibers), synthetic fibers (e.g., polymeric fibers such as polyester, polypropylene, or polyethylene fibers) or from a combination of natural and synthetic fibers.

Suitable nonwoven materials include fibrous nonwoven materials formed by a carding process or a spunbond process or meltblown process whereby molten polymeric material is extruded through a die, attenuated to lengthen the extruded polymer into fibers and decrease the diameter thereof and is subsequently deposited on a forming surface. Methods of forming such nonwoven materials are known to those skilled in the art. Polymeric materials suitable for use in forming such fibrous nonwoven materials include polyolefins such as polyethylene and polypropylene, polyesters, nylons, ethylene vinyl acetate, ethylene methacrylate, copolymers of the above materials, block copolymers such as A-B-A block copolymers of styrene and butadiene, and the like.

Beside the conventional nonwoven materials described herein before, the apertured topsheet for use herein can be made of conventional nonwoven materials typically provided by any process mentioned herein before like spunbond process, into which additional apertures can be formed by any conventional method known to those skilled in the art for this purpose after the nonwoven materials have been formed per se.

In some embodiments wherein the topsheet comprises an upper layer and a lower layer, at least one of the upper and lower layers, preferably the upper layer comprises a liquid permeable apertured polymeric film. Suitable apertured polymeric films include the apertured polymeric films hereinbefore disclosed.

In some embodiments, the upper layer is provided by a film material having apertures which are provided to facilitate liquid transport from the wearer facing surface towards the absorbent core.

In some of the embodiments wherein the topsheet comprises an upper and a lower layer, the lower layer preferably comprises a non woven layer, an apertured formed film or an airlaid tissue. Suitable formed films include the apertured formed films hereinbefore disclosed.

In some embodiments, the topsheet is an apertured topsheet which comprises an apertured polymeric film or an apertured formed film together with a nonwoven layer, the nonwoven layer being directed towards the wearer surface. Typically the nonwoven layer is disposed onto the apertured polymeric film or apertured formed film in a discontinuous way so that at least the region where liquid is expected to be discharged onto the absorbent article is free of the nonwoven layer.

In some embodiments, the topsheet is a so-called hybrid topsheet in which the wearer contacting surface is provided in its longitudinal center by an apertured polymeric film while a region not including the center is provided with a non-woven such as e.g. the high loft non-woven or other non-woven which does provide particularly skin friendliness. Such topsheets have been disclosed in EPA-523 683, EP-A-523 719, EP-A-612 233, or EP-A-766 953.

### The backsheet

The backsheet primarily prevents the body exudates absorbed and contained in the absorbent core from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions. In some embodiments, the backsheet comprises a first layer which provides the garment facing surface of the backsheet and a second layer (also called secondary backsheet) between the first layer and the absorbent core.

In some embodiments, the backsheet is preferably breathable in addition to the prevention of liquid transport through the backsheet. Hence, the backsheet also permits the transfer of water vapour and preferably both water vapour and air through it and thus allows reduction of humid and occlusive environment on the skin contacted with the article. Breathability of the backsheet contributes to further improve the freshness sensation and dry feeling associated with the cooling agent used according to the present invention.
In some embodiments, the disposable absorbent article comprises both a breathable backsheet and an apertured polymeric film topsheet for further improved freshness sensation of the article comprising the composition of the present invention.

Suitable breathable backsheets for use herein include all breathable backsheets known in the art. In principle there are two types of breathable backsheets, single layer breathable backsheets which are breathable and impervious to liquids and backsheets having at least two layers, which in combination provide both breathability and liquid imperviousness.

Suitable single layer breathable backsheets for use herein include those described for example in GB A 2184 389, GB A 2184 390, GB A 2184 391, US 4 591 523, US 3 989 867, US 3 156 242 and WO 97/24097.

Suitable dual or multi layer breathable backsheets for use herein include those exemplified in US 3 881 489, US 4 341 216, US 4 713 068, US 4 818 600, EP 203 821, EP 710 471, EP 710 472, WO 97/24095, WO 97/24096, WO 97/24097 and EP 793 952.

In addition to the components described herein above, the absorbent article may also comprise all those features and parts which are typical for products in the context of their intended use such as wings and side flaps, undergarment adhesive means, release paper, wrapping elements, fastening means and the like. Optionally, the absorbent article can comprise all those components typical for the intended product use. For example, the absorbent article can comprise components such as wings in order to improve the positioning and soiling protection performance especially towards the rear end of the article. Such designs are shown for example in EP 130 848 or EP 134 086, Thong liners with wings are shown in US design 394,503, UK designs 2,076,491 and 2,087,071 as well as internationally filed industrial model DM 045544, filed under the Hague Agreement, registered on October 21, 1998.

Irrespective whether the wings are specially designed for thong liners or for conventional absorbent articles they can be provided as separate pieces and be attached to the thong liner or conventional pantiliners or sanitary napkins, or they can be integral with the materials of the absorbent articles, e.g. by being integral extension of the topsheet, the backsheet or a combination thereof. If the wings are attached then they can be attached in a basic outward pointing position or already be predisposed towards their in-use position, i.e. towards the longitudinal centerline.

The absorbent article can also comprise a fastening adhesive for attachment. In the case of sanitary napkins, pantliners or thong liners a so-called panty fastening adhesive can be present on the backsheet for attachment to an undergarment.

In some embodiments, the absorbent article may be a diaper, a training pant, a haemorrhoidal article or an adult incontinence undergarment.

In some other embodiments, the absorbent article may be an absorbent article for feminine hygiene. In some of these embodiments, the absorbent article may be a sanitary napkin, a pantyliner, vaginal tampon or an interlabial pad.

An absorbent article comprising a composition according to the present invention comprises a sufficient amount of the cooling agent to stimulate the thermo-receptors in the areas of the skin and/or mucosal surfaces with which the article comes into contact and thereby convey the desired freshness sensation.

In some embodiments, the absorbent article comprises from 5 to 500 mg, or 10 to 300 mg or 15 to 200 mg of cooling agent per absorbent article.

In some embodiments, the absorbent article comprises from 4 to 200 gsm (grams per square meter), or 10 to 100 gsm, or 20 to 100 gsm of the composition according to the present invention. Grams per square meter are derived from the mass of composition divided by the area on which the composition is applied.

In the embodiments wherein the absorbent article comprises a topsheet, a backsheet and an absorbent core which is positioned between the topsheet and the backsheet, the composition may be comprised by the topsheet and/or the backsheet and/or the absorbent core of the absorbent article.

When released from one of the elements of the absorbent article: topsheet, backsheet or absorbent core, the agent is free to migrate from the location of the element to the skin and/or mucosal surfaces of the wearer of the absorbent article.

In some embodiments, wherein the absorbent article is a diaper, a training pant or an adult incontinence undergarment, the composition may also be comprised by additional elements of the absorbent article (instead of or in addition to the above-mentioned elements) such as the leg cuff(s) and/or side panel(s) and/or waist region(s) of the absorbent article.

In some embodiments, wherein the absorbent article is a sanitary napkin, the composition may also be comprised by additional elements of the absorbent article (instead of or in addition to the above-mentioned elements) such as the wings of the sanitary napkin.

In some embodiments, the composition is comprised by a topsheet comprising an upper layer which provides the user facing surface of the topsheet and a lower layer positioned between the upper layer and the absorbent core, as described hereinbefore. In such embodiments, the composition may be comprised either by the upper layer or the lower layer or both layers. However, it is particularly advantageous to have the composition which is comprised by the lower layer since in such a configuration, the composition is in close proximity with the skin of the wearer which facilitates the transfer of the agent to the skin of the wearer but not in direct contact with the skin of the wearer which therefore reduces the risk of skin irritation that could eventually occur when the composition is in contact with the skin of the wearer.

In the embodiments, wherein the composition is comprised by the absorbent core of the absorbent article, the composition may either be comprised by the primary and/or secondary fluid distribution layer (when present) and/or the fluid storage layer and/or the "dusting" layer when present.

The composition according to the present invention will have in general the form of a waxy solid which turns into a liquid upon heating. The composition can be applied onto the element(s) of the absorbent article in any way which will be apparent to a person skilled in the art, for example by using an equipment for hot melt glue application, by printing or by soaking the element in the composition.

In some embodiments, the composition is heated before application onto the element(s) of the absorbent article to a temperature of from 50°C to 100°C to facilitate the application of the composition to the absorbent article.

The composition can be applied to the absorbent article in various defined patterns such as dot(s), stripe(s), square(s), circle(s), or oval(s). In the embodiments wherein the composition is applied to the absorbent article in a stripe(s) pattern, the length of each stripe can be up to the length of the absorbent article and the width can be from 0.1 mm to 50 mm, or 0.5 mm to 20 mm, or 1 mm to 10 mm.

In some embodiments, the composition is applied to specific regions of the absorbent article as for example disclosed in WO 03/051260 A1.

In some embodiments, the composition of the present invention may be comprised by an insertable element such as an absorbent material or a strip of material which is inserted into the absorbent article for use during wear of the article. The insertable element is typically positioned in proximity to the wearer's skin.

In some embodiments, the composition may be comprised by a delivery system for example, pressure-rupturable or dissolvable microcapsule(s) that are induced to express the composition upon dissolving due to contact with moisture from body exudates, or rupturing due to pressure from the body or manual rupturing by a user prior to applying the article to a wearer. For example, a water-soluble film that encloses and expresses a powder upon contact with moisture is described in U.S. Patent No. 4,790,836 and would be a suitable material for use in microcapsules containing the composition. Examples of pressure-rupturable microcapsules suitable for containing the agent are described in U.S. Patent 3,585,998. Such microcapsules may be present in any portion of the article, including the wearer-facing surface. U.S. Patent 4,623,339 describes an insertable layer that is removable from an article prior to use and manually pressure activatable to express a substance through slits in the layer.

Other suitable delivery systems for containing the composition include, but are not limited to, "cells" in the article that are enclosed or partially enclosed voids, regularly or irregularly shaped, that release the composition when in contact with moisture, heat or pressure; and water-soluble adhesives and other such compositions which release the composition upon contact with moisture, and the like.

In all the above-mentioned configurations of the absorbent article comprising the composition according to the present invention, the cooling agent is activated by contact with moisture from bodily exudates such as urine, sweat, feces or menses. Indeed, when bodily exudates enter in contact with the composition of the present invention, PEGs which are comprised within the composition are dissolved by the bodily exudates. This enables the release of the cooling agent from the composition.

### EXAMPLES

### Compositions

Three different compositions were prepared and compared according to the Comparative Oven Test described below in order to determine the stability of menthyl lactate in the compositions.

### • Comparative Example 1

The composition of the comparative example 1 only comprises menthyl lactate which is available from Symrise GmbH & Co. KG, Muhlenfeldstrasse 1, D-37603 Holzminden under the tradename Frescolat ML®.

### • Comparative Example 2

The composition of the comparative example 2 is a composition which comprises the same menthyl lactate as in Comparative Example 1 and PEG 1500 available from Clariant prodotti (Italia) S.p.A, Via Manzoni 37, 20030 Palazzolo Milanese (MI) under the Trade name polyglykol 1500 S.

70 g of PEG 1500 are melted at 80°C in a 250 ml beaker. Then, heating is stopped and 30 g of menthyl lactate are added under stirring with a magnetic stirrer until the crystals of menthyl lactate are melted and the mixture is homogeneous. Then, the mixture is let solidify at ambient temperature.

### • Example 1

The composition of the Example 1 is a composition which comprises the same menthyl lactate as in Comparative Example 1 and PEG 20000 available from Clariant prodotti (Italia) S.p.A, Via Manzoni 37, 20030 Palazzolo Milanese (MI) under the Trade name polyglycol 20000 S.

70 g of PEG 20000 are melted at 80°C in a 250 ml beaker. Then, heating is stopped and 30 g of menthyl lactate are added under stirring with a magnetic stirrer until the crystals are melted and the mixture is homogeneous. Then, the mixture is let solidify at ambient temperature.

### Absorbent article

90 mg of a composition according to Example 1 was applied with a Meltex EP45 hot melt applicator having a head operating temperature of about 90°C with a 6 stripe 1 mm wide pattern across the longitudinal surface of the wearer-facing surface of the topsheet of a feminine pad Always™ Regular as sold by The Procter & Gamble Company.

### Comparative Test - Oven Test

3 x 25 ml Pyrex® beakers are provided (diameter 3 cm, height 4.5 cm) and numbered 1-3. 0.5 g of cooling agent from comparative example 1 (menthyl lactate) are weighed into beaker number 1. 1.67 g of the composition of comparative example 1 (containing 0.5 g of menthyl lactate) are weighed into beaker number 2. 1.67 g of the composition of Example 1 (containing 0.5 g of menthyl lactate) are weighed into beaker number 3.

The three beakers are put into a ventilated precision oven Memmert UFE 400 model. The temperature is set at 40.0 °C and air ventilation regulated to have a mean air speed of 0.60 m/s and a volume of aspired air of 1.96 m³/h.

The beakers are kept in the oven for 60 days and then weighed. The weight loss is due to the evaporation of menthyl lactate as PEG is not volatile. The weight percentages of menthyl lactate loss for each beaker are summarized in Table 1 below.

**Table 1**

| | menthyl lactate loss |
|---|---|
| Beaker 1 (Comparative Example 1) | 100% |
| Beaker 2 (Comparative Example 2) | 80% |
| Beaker 3 (Example 1) | 30% |

## Claims

1. A composition comprising:
a) a cooling agent, wherein the cooling agent is selected from the menthol derivatives group consisting of menthol, menthyl lactate, menthone glycerin acetal and combinations thereof and
b) a polyethylene glycol or a mixture of different polyethylene glycols, wherein the polyethylene glycol(s) has/have a weight average molecular weight M_{w} of 5000 to 40000 Da.

2. The composition according to claim 1, wherein the weight average molecular weight(s) M_{w} of the polyethylene glycol(s) is/are of 10000 to 30000 Da

3. The composition according to any of the preceding claims, wherein the weight percentage of cooling agent in the composition is of 1 to 99 wt%.

4. The composition according to any of the preceding claims, wherein the weight percentage of cooling agent in the composition is of 10 to 70 wt%.

5. An absorbent article comprising the composition according to any of the preceding claims.

6. The absorbent article according to claim 5 comprising from 5 to 500 mg of cooling agent.

7. The absorbent article according to claim 5, comprising from 10 to 300 mg of cooling agent.

8. The absorbent article according to claims 5 to 7, wherein the absorbent article is a diaper, a training pant, a haemorrhoidal article, or an adult incontinence undergarment.

9. The absorbent article according to claims 5 to 7, wherein the absorbent article is an absorbent article for feminine hygiene selected from the group consisting of sanitary napkins, pantyliners, vaginal tampons or interlabial pads.

10. The absorbent article according to claims 5 to 9, wherein the absorbent article comprises a topsheet, a backsheet and an absorbent core, the absorbent core being positioned between the topsheet and the backsheet and wherein the composition is comprised by any of the elements selected from the group consisting of the topsheet, the backsheet, the absorbent core, and combinations thereof.

11. The absorbent article according to claim 10, wherein the topsheet comprises an upper layer facing the user in use and a lower layer positioned between the upper layer and the absorbent core, wherein the composition is comprised by the lower layer of the topsheet.

12. The absorbent article according to claims 1 to 5, wherein the composition is comprised by an insertable element which is inserted into the article during use of the article.

## Patentansprüche

1. Zusammensetzung, umfassend:
a) ein Kühlmittel, wobei das Kühlmittel ausgewählt ist aus der Mentholderivategruppe bestehend aus Menthol, Menthyllactat, Menthonglycerinacetal und Kombinationen davon, und
b) ein Polyethylenglykol oder eine Mischung verschiedener Polyethylenglykole, wobei das bzw. die Polyethylenglykol(e) ein durchschnittliches Molekulargewicht (Gewichtsmittel) M_{G} von 5000 bis 40000 Da oder mehr aufweist bzw. aufweisen.

2. Zusammensetzung nach Anspruch 1, wobei das bzw. die durchschnittliche(n) Molekulargewicht(e) (Gewichtsmittel) M_{G} des bzw. der Polyethylenglykol(e) 10000 bis 30000 Da beträgt bzw. betragen.

3. Zusammensetzung nach einem der vorstehenden Ansprüche, wobei der Prozentanteil an Kühlmittel in der Zusammensetzung 1 bis 99 Gew.-% beträgt.

4. Zusammensetzung nach einem der vorstehenden Ansprüche, wobei der Prozentanteil an Kühlmittel in der Zusammensetzung 10 bis 70 Gew.-% beträgt.

5. Absorptionsartikel, umfassend die Zusammensetzung nach einem der vorstehenden Ansprüche.

6. Absorptionsartikel nach Anspruch 5, umfassend 5 bis 500 mg Kühlmittel.

7. Absorptionsartikel nach Anspruch 5, umfassend 10 bis 300 mg Kühlmittel.

8. Absorptionsartikel nach einem der Ansprüche 5 bis 7, wobei der Absorptionsartikel eine Windel, eine Übungshose, ein Hämorrhoidalartikel oder ein Inkontinenzunterwäscheartikel für Erwachsene ist.

9. Absorptionsartikel nach Anspruch 5 bis 7, wobei der Absorptionsartikel ein Absorptionsartikel für die Damenhygiene ist, der ausgewählt ist aus der Gruppe bestehend aus Damenbinden, Slipeinlagen, Vaginaltampons oder Interlabialeinlagen.

10. Absorptionsartikel nach Anspruch 5 bis 9, wobei der Absorptionsartikel eine Oberschicht, eine Unterschicht und einen Absorptionskern umfasst, wobei der Absorptionskern zwischen der Oberschicht und der Unterschicht angeordnet ist und wobei die Zusammensetzung in einem der Elemente enthalten ist, die ausgewählt sind aus der Gruppe bestehend aus der Oberschicht, der Unterschicht, dem Absorptionskern und Kombinationen davon.

11. Absorptionskern nach Anspruch 10, wobei die Oberschicht eine obere Schicht, die während des Gebrauchs zum Benutzer zeigt, und eine untere Schicht umfasst, die zwischen der oberen Schicht und dem Absorptionskern angeordnet ist, wobei die Zusammensetzung in der unteren Schicht der Oberschicht enthalten ist.

12. Absorptionsartikel nach Anspruch 1 bis 5, wobei die Zusammensetzung in einem einführbaren Element enthalten ist, das während des Gebrauchs des Artikels in den Artikel eingeführt wird.

## Revendications

1. Composition comprenant :
a) un agent de refroidissement, dans laquelle l'agent de refroidissement est choisi parmi le groupe de dérivés du menthol constitué de menthol, lactate de menthyle, menthone glycérine acétal et leurs combinaisons ; et
b) un polyéthylène glycol ou un mélange de différents polyéthylène glycols, dans laquelle le(s) polyéthylène glycol(s) a/ont un poids moléculaire moyen en poids M_{w} de 5 000 à 40 000 Da.

2. Composition selon la revendication 1, dans laquelle le(s) poids moléculaire(s) moyen(s) en poids M_{w} du(des) polyéthylène-glycol(s) est/sont compris entre 10 000 et 30 000 Da.

3. Composition selon l'une quelconque des revendications précédentes, dans laquelle le pourcentage en poids de l'agent de refroidissement dans la composition est compris entre 1 et 99 % en poids.

4. Composition selon l'une quelconque des revendications précédentes, dans laquelle le pourcentage en poids de l'agent de refroidissement dans la composition est compris entre 10 et 70 % en poids.

5. Article absorbant comprenant la composition selon l'une quelconque des revendications précédentes.

6. Article absorbant selon la revendication 5, comprenant entre 5 et 500 mg d'agent de refroidissement.

7. Article absorbant selon la revendication 5, comprenant entre 10 et 300 mg d'agent de refroidissement.

8. Article absorbant selon les revendications 5 à 7, dans lequel l'article absorbant est une couche, une culotte d'apprentissage, un article pour hémorroïdes ou un sous-vêtement d'incontinence pour adulte.

9. Article absorbant selon les revendications 5 à 7, dans lequel l'article absorbant est un article absorbant pour l'hygiène féminine sélectionné dans le groupe composé de serviettes hygiéniques, protège-slip, tampons vaginaux ou tampons interlabiaux.

10. Article absorbant selon les revendications 5 à 9, dans lequel l'article absorbant comprend une feuille supérieure, une feuille arrière et un coeur absorbant, le coeur absorbant étant positionné entre la feuille supérieure et la feuille arrière et dans lequel la composition est composée par l'un quelconque des éléments sélectionnés dans le groupe composé de la feuille supérieure, la feuille arrière, le coeur absorbant et des combinaisons de ces éléments.

11. Article absorbant selon la revendication 10, dans lequel la feuille supérieure comprend une couche de dessus faisant face à l'utilisateur en utilisation et une couche de dessous positionnée entre la couche de dessus et le coeur absorbant, dans lequel la composition est composée par la couche de dessous de la feuille supérieure.

12. Article absorbant selon les revendications 1 à 5, dans lequel la composition est composée d'un élément insérable, qui est inséré dans l'article lorsque celui-ci est utilisé.
